# FASCICULE DE BREVET EUROPEEN

(11) **EP 2 015 764 B1**
(45) Date de publication et mention de la délivrance du brevet: **18.11.2015**
(21) Numéro de dépôt: 07731427.6
(22) Date de dépôt: 09.05.2007
(51) Int. Cl.: A61K 36/81, A61P 25/34

(54) **EXTRAIT AQUEUX DE FEUILLES DE TABAC , SES UTILISATIONS DANS LE TRAITEMENT DE LA DEPENDANCE**
WÄSSRIGER TABAKBLATT-EXTRAKT, SEINE VERWENDUNGEN BEI DER BEHANDLUNG VON ABHÄNGIGKEIT
AQUEOUS TOBACCO LEAF EXTRACT, USES THEREOF IN THE TREATMENT OF DEPENDENCE

(30) Priorité: 09.05.2006 FR 0604104
(43) Date de publication de la demande: 21.01.2009
(73) Titulaire: NFL Biosciences, 75006 Paris (FR)
(72) Inventeur: NICOLAS, Jean-Pierre, F-84290 Sainte Cécile Les Vignes (FR)
(74) Mandataire: Regimbeau
(86) Numéro de dépôt international: PCT/FR2007/000786
(87) Numéro de publication internationale: WO 2007/128924

(56) Documents cités:
- EP-A1- 1 304 048
- IE-A1- 960 511
- US-A1- 2003 185 908
- ANTHENELLI ET AL: "Recent advances in the treatment of tobacco dependence" CLINICAL NEUROSCIENCE RESEARCH, ELSEVIER, LONDON, GB, vol. 5, no. 2-4, novembre 2005 (2005-11), pages 175-183, XP005149967 ISSN: 1566-2772

## Description

La présente décrit l'utilisation d'un extrait de feuilles de tabac pour la préparation d'un médicament destiné au traitement de la dépendance. L'invention a également pour objet une solution et un kit pour injection comprenant des extraits de feuilles de tabac.

La dépendance ou addiction a été définie par l'Organisation Mondiale de la Santé comme « un syndrome pour lequel la consommation d'un produit devient une exigence supérieure à celles d'autres comportements qui avaient auparavant une plus grande importance. Dans sa forme extrême l'état de dépendance se caractérise par un besoin irrésistible d'un produit qui pousse l'individu souffrant de cette dépendance à la recherche impulsive de ce produit ».

En France, on considère qu'il y aurait environ 200 000 individus sous la dépendance de l'héroïne et beaucoup moins sous la dépendance de la cocaïne ou des dérivés de l'amphétamine. Cependant d'autres produits, dont les effets sont moins remarqués entraînent une dépendance : alcool, tabac, café. Cette dépendance entraîne également des problèmes de santé publique importants.

Ainsi, en particulier contre la dépendance tabagique, sans parler des psychothérapies comportementales ou de l'acupuncture, il existe actuellement trois grands types de traitement de la dépendance tabagique :
- les substituts nicotiniques ;
- le Zyban® ;
- l'homéopathie.

Le principe d'action de ces méthodes est fondé sur l'affirmation que la nicotine est impliquée dans le mécanisme de la dépendance tabagique. Cette molécule peut se fixer à des protéines présentes à la surface des cellules nerveuses, les récepteurs nicotiniques à l'acétylcholine. En présence de nicotine, ces récepteurs qui sont en fait des canaux s'ouvrent. S'en suit alors une cascade d'événements qui aboutissent à la libération d'une hormone, la dopamine. La nicotine stimule "le circuit de la récompense" et procure ainsi une sensation de satisfaction.

Lors du sevrage tabagique, le corps "réclame" sa dose de nicotine pour satisfaire cette sensation de bien être : c'est le manque.

Les substituts nicotiniques ont donc pour objet d'apporter au cerveau du fumeur une quantité de nicotine suffisante pour lui éviter les symptômes de manque.

Les substituts nicotiniques peuvent être administrés de diverses manières, par voie transdermique sous forme de patchs ou timbres, par voie orale sous forme de gommes à mâcher, comprimés à sucer ou comprimés sublinguaux, ou par voie aérienne sou forme d'inhalateur. Les formes orales peuvent être utilisées seules ou associées ponctuellement au patch. Les patchs ou timbres délivrent de la nicotine qui est facilement absorbée par la peau ce qui permet de soulager les symptômes de sevrage physique en rapport avec le manque de nicotine.

L'utilisation de patchs est recommandée par les experts du Ministère de la Santé. Même si la motivation est le facteur essentiel du succès, on considère que l'efficacité du sevrage tabagique est doublée par rapport à un placebo après pose de patchs. Au total, 16 à 20 % environ des fumeurs parviennent à s'arrêter de fumer après un an grâce à cette aide.

Les gommes à mâcher (ou "chewing-gum") à la nicotine permettent un apport de nicotine sous forme buccale qui soulage les symptômes de manque physique. Le nombre de gommes à mâcher peut être modifié en fonction du niveau de dépendance pharmacologique, lequel est évalué comme pour les patchs par les réponses au test de Fagerstrôm. La consommation de gommes à mâcher dure en général trois mois et il est recommandé de ne plus les utiliser au-delà de six mois après l'arrêt du tabac.

Plusieurs essais thérapeutiques ont démontré que l'efficacité de ces gommes est comparable à celle des patchs, avec un taux de sevrage de 19 % à un an.

Pour obtenir un effet optimal, la dose et la durée du traitement doivent être suffisantes et le mode d'utilisation doit être respecté (en les suçant au départ puis en les mâchant très lentement pendant 30 à 40 minutes). En effet, si la mastication est trop rapide, la nicotine diffuse trop vite et risque de provoquer une hyper salivation et parfois des brûlures gastriques ou un hoquet. Par ailleurs, la gomme est en partie inefficace lorsqu'elle est avalée car elle est détruite en grande partie dans le foie. Certains ex-fumeurs semblent, par ailleurs, éprouver des difficultés à se passer des gommes.

Les gommes peuvent être employées en complément du timbre pour calmer une envie brusque de fumer, non couverte par celui-ci. Elles sont bien adaptées au cas des personnes qui fumaient de façon irrégulière et permettent aux ex-fumeurs de jouer un rôle actif dans le sevrage. Elles assurent aussi le maintien d'une certaine gestuelle.

La nicotine peut également être fournie sous forme de comprimés à déposer sous la langue ou à sucer.

L'utilisation de ces comprimés est plus discrète et plus facile que celle des gommes à mâcher.

Comme les autres substituts nicotiniques, ces comprimés peuvent induire des maux de tête en début de traitement.

La nicotine peut également être apportée par inhalation. L'inhalateur se compose d'un embout avec une cartouche qui ressemble à un porte-cigarettes et délivre de la nicotine sous forme d'inhalations buccales. En cas d'envie de fumer, l'ex-fumeur inhale une bouffée qui lui fournit environ 5 mg de nicotine.

L'inhalateur permet non seulement de soulager les symptômes de manque en rapport avec l'absence de nicotine, mais aussi d'agir sur la gestuelle en mimant l'acte de fumer. Il peut, comme les gommes à mâcher et les comprimés sublinguaux ou à sucer, être utilisé en complément d'un timbre.

Les concentrations sanguines de nicotine sont plus lentes à obtenir que lorsqu'on fume une cigarette et le fumeur doit donc attendre pour que cesse son envie de fumer.

Une autre méthode de sevrage consiste en l'administration de bupropion, commercialisé sous la marque Zyban® par les Laboratoires GlaxoSmithKline qui agit sur certains neuromédiateurs cérébraux comme les catécholamines, la noradrénaline et la dopamine. ZYBAN® est un inhibiteur sélectif de la recapture neuronale des catécholamines, ce qui lui confère des propriétés d'antidépresseur. Ce médicament, qui est également commercialisé depuis 1989 aux États-Unis pour ses propriétés anti-dépressives, permet de diminuer certains symptômes associés au sevrage comme l'envie de fumer et les difficultés de concentration.

L'efficacité de Zyban® est équivalente à celle obtenue après pose de timbres nicotiniques (taux de sevrage autour de 20 %). Zyban® a également démontré dans des études cliniques une bonne activité chez les bronchitiques chroniques, des malades souvent gros fumeurs qui ont généralement du mal à se débarrasser du tabac.

Zyban® agit sur la composante psychique de la dépendance au tabac et facilite le sevrage tabagique par un mécanisme différent des substituts nicotiniques.

Zyban® nécessite une prescription médicale et peut entraîner une sensation de bouche sèche, d'insomnies et de vertiges. Ce médicament a fait l'objet de mesures de surveillance de pharmacovigilance de la part de l'Agence française de sécurité sanitaire des produits de santé (Afssaps) car des décès ont été observés en Grande-Bretagne après son administration. Néanmoins, les réactions graves de ce médicament semblent être rares, lorsque Zyban® a été correctement prescrit et ses contre-indications respectées.

La troisième voie de sevrage est l'homéopathie qui repose sur l'utilisation à doses infinitésimales, obtenues grâce à des dilutions successives, de la substance provoquant les symptômes que l'on désire combattre. C'est pourquoi un extrait de "tabacum" est souvent utilisé dans le sevrage tabagique. La demande de brevet irlandaise IE 960 511 décrit notamment l'utilisation de dilutions homéopathiques d'extrait de tabac pour la fabrication d'un médicament destiné à la restauration des fonctions neuronales.

Ses effets ne sont pas démontrés dans le sevrage tabagique. Comme les autres techniques non conventionnelles de sevrage, son efficacité n'est pas suffisante chez les gros fumeurs.

Pour les experts du ministère de la santé, l'utilisation d'extrait de "tabacum", c'est-à-dire d'extraits aqueux de feuilles de tabac, à des dosages très faibles (inférieurs à 0,00001g/ml.) est justifiée uniquement dans le traitement des allergies au tabac, lesquelles sont tout à fait exceptionnelles. L'extrait est alors administré par cure de mésothérapie selon un protocole très précis.

Les mécanismes de dépendance ou d'addiction sont complexes et non encore totalement élucidés. Cependant, des travaux récents ont mis en évidence que la dépendance implique la participation de trois neuromodulateurs, la dopamine, la noradrénaline et la sérotonine (Jean-Paul Tassin et Jacques Glowinski dans Comptes rendus de l'Académie des sciences américaines, 24 avril 2006). Le dérèglement de la cinétique de production de ces neuromodulateurs reflète l'installation de la dépendance.

Classiquement, la nicotine a été considérée comme la seule composante de la cigarette induisant une dépendance et de ce fait l'essentiel des méthodes de sevrage tabagique repose sur la prise de nicotine. Cette approche est désormais remise en question, la nicotine n'apparaissant pas comme étant un facteur essentiel de la dépendance selon les travaux récents précédemment cités.

De façon surprenante et inattendue le présent inventeur a trouvé que l'injection d'une solution aqueuse d'un extrait aqueux de feuilles de tabac permettait de réduire la dépendance. Plus particulièrement, les inventeurs ont pu démontrer qu'une unique injection d'un extrait de tabac selon l'invention était généralement suffisante pour réduire voire supprimer la dépendance tabagique des fumeurs. Cette caractéristique constitue un avantage majeur pour le patient, étant donné que les produits actuellement disponibles sur le marché ne prévoient que des traitements à long terme et en plusieurs prises. A l'inverse, le traitement selon l'invention propose une thérapie de choc, c'est-à-dire un traitement constitué de préférence d'une seule injection d'un extrait de tabac, optionnellement suivie, après plusieurs jours voire semaines, d'au moins une deuxième injection si le sujet traité en ressent le besoin.

Selon un premier objet, la présente invention porte sur une solution stérile aqueuse telle que définie à l'une des revendications 1 ou 2.

Selon un autre objet, l'invention, porte sur un kit tel que défini à l'une des revendications 3 ou 4.

Selon un troisième objet, l'invention porte sur une solution pour une utilisation selon l'une quelconque des revendications 5 à 10.

L'invention a également pour objet l'utilisation d'un extrait aqueux de feuilles de tabac tel que revendiqué pour la préparation d'un médicament pour le traitement de la dépendance.

Plus particulièrement, l'invention a pour objet l'utilisation d'un extrait aqueux de feuilles de tabac pour la préparation d'un médicament sous la forme d'une solution dans de l'eau stérile pour injection, de préférence pour une administration par voie sous cutanée, pour le traitement de la dépendance.

L'extrait aqueux de feuilles de tabac comprend de nombreux composés, mais ne contient que de très faibles quantités de nicotine, il peut même être sensiblement dépourvu de nicotine.

Sans vouloir être lié par une théorie, l'inventeur est d'avis que les substances présentes dans les extraits aqueux de feuilles de tabac agissent contre le découplage pathologique des trois principaux neuromodulateurs, à savoir la dopamine, la noradrénaline et la sérotonine.

Selon un mode de réalisation avantageux, la présente invention concerne l'utilisation d'un extrait aqueux de feuilles de tabac pour la préparation d'un médicament pour le traitement de la dépendance tabagique.

De façon avantageuse, on utilisera des lyophilisats d'extraits aqueux de feuilles de tabac. En effet, l'utilisation d'extraits aqueux de feuilles de tabac permet d'avoir disponibles à toute période de l'année des extraits aqueux de feuilles de tabac sans que leur disponibilité soit liée à la saison de la cueillette du tabac.

Selon un mode de réalisation avantageux, le médicament se présente sous la forme d'une injection pour une administration unique, de préférence par voie sous cutanée.

L'administration d'extraits de feuilles de tabac a pour conséquence de réduire durablement (3 à 5 semaines) et très fortement voire totalement le niveau des exigences de l'organisme induisant de ce fait une suppression de l'envie de reprendre la substance toxique dont le patient est dépendant, en particulier la cigarette, et des stress associés. Ainsi, une seule injection est généralement suffisante pour réduire voir supprimer les symptômes de la dépendance tabagique. Evidemment, au moins une injection ultérieure supplémentaire peut être nécessaire en fonction du patient traité, en particulier en fonction de son niveau de dépendance tabagique. L'homme du métier est à même d'adapter l'intervalle de temps entre la première et la seconde injection en fonction de chaque patient, et éventuellement le nombre supplémentaire d'injections.

Sans vouloir être lié par une théorie, l'inventeur est d'avis que ce mode d'administration permet aux substances actives présentes dans l'extrait de feuilles de tabac de se trouver très rapidement dans la circulation sanguine et de permettre la libération massive des neuromodulateurs impliqués dans les mécanismes de la dépendance. On peut alors parler d'un mécanisme à effet de seuil obtenu par saturation et neutralisation rémanentes des sites inducteurs des circuits de stress. Bien entendu, tout mode d'administration qui permettrait un effet de seuil du même ordre serait approprié. Ainsi, une administration par voie intramusculaire ou par voie intraveineuse serait envisageable.

La désintoxication est ipso facto possible et entamée. Elle se poursuivra durant toute la période de rémanence induite par le traitement.

La tolérance est parfaite, sans risque de développement d'une quelconque addiction.

Selon un mode de réalisation particulier, l'invention porte sur l'utilisation d'un extrait aqueux de feuilles de tabac à une teneur en matière sèche, exprimée en mg /ml, de 0,05 à 150 mg de matière sèche par ml d'eau stérile, de préférence de 0,5 à 100mg par ml d'eau stérile, et plus préférentiellement encore de 1 à 20mg par ml d'eau stérile, pour la préparation d'une solution pour injection pour le traitement de la dépendance.

Même à des concentrations élevées, les extraits aqueux de feuilles de tabac ne s'avèrent pas toxiques, notamment lorsqu'ils sont administrés par voie sous-cutanée. Cependant, en utilisant une teneur supérieure à 150 mg/ml l'efficacité du traitement n'a pas été améliorée. En revanche, au dessous d'une teneur de 0,05 mg/ml, les résultats en termes d'efficacité du traitement étaient moindres.

Selon le degré de dépendance du patient et son aptitude à contrôler les stress liés, une seule injection pourra s'avérer suffisante.

L'invention a donc pour objet l'utilisation d'un extrait aqueux de feuilles de tabac pour la préparation d'une injection unique pour le traitement de la dépendance.

En effet, notamment dans le cas de la dépendance tabagique, il s'est avéré qu'une seule injection d'extraits aqueux de feuilles de tabac était suffisante pour que le patient soit affranchi des manifestations de la dépendance, c'est-à-dire notamment perturbations du sommeil, excitabilité, nervosité, tonicité hyperactive. Dès la première injection, le patient voit apparaître les signes caractéristiques annonçant un succès de la désintoxication : sommeil profond, fatigabilité dès les premiers jours, récupération progressive et rapide de l'odorat, du goût, de la sensibilité de la gorge.

Dans d'autres cas, une seule injection n'est pas suffisante, il est alors nécessaire d'en pratiquer au moins une seconde.

L'invention a donc également pour objet, l'utilisation d'un extrait aqueux de feuilles de tabac pour la préparation d'au moins deux injections faites à des intervalles de temps de 4 à 30, de préférence 5 à 15, plus préférentiellement encore de 6 à 13 jours.

La nécessité de pratiquer une seconde injection, et éventuellement d'autres injections supplémentaires, sera déterminée pour chaque patient en fonction des résultats obtenus suite à la première injection et/ou suite aux injections précédentes.

Dans le cas de la dépendance tabagique, le nombre d'injections ne devrait pas être supérieur à 5, de préférence pas supérieur à 4, et plus préférentiellement encore pas supérieur à 2.

Suite à cette injection ou ces injections, le patient ressent une forte motivation pour assurer de lui-même le succès total de la désintoxication.

Bien entendu, pour une efficacité optimale, ces injections doivent s'accompagner des recommandations classiques à faire lors d'un sevrage, c'est-à-dire qu'il est fortement conseillé d'éviter systématiquement la consommation d'alcool, d'épices fortes, de café car il est reconnu que l'envie de reprendre une cigarette, est souvent associée à ces goûts et entraîne un réflexe dit de Pavlov.

L'invention porte également sur des solutions aqueuses stériles d'extraits aqueux de feuilles de tabac à une teneur en matière sèche de 0,05 à 150mg/ml, de préférence de 0,5 à 100mg/ml, et plus préférentiellement encore de 1 à 20 mg/ml d'eau stérile.

De façon avantageuse, les extraits aqueux sont lyophilisés.

Dans un mode de réalisation particulier, l'invention concerne également des solutions stériles aqueuses d'un extrait aqueux de feuilles de tabac à une teneur en matière sèche de 0,05 à 50 mg/ml d'eau stérile, de préférence de 0,5 à 20 mg/ml d'eau stérile, et plus préférentiellement encore de 1 à 15 mg/ml d'eau stérile, de préférence ledit extrait étant lyophilisé.

L'invention porte également sur un kit prêt-à-l'emploi comprenant une seringue, un flacon rempli d'eau stérile et un lyophilisat d'extrait aqueux de feuilles de tabac.

Ces solutions aqueuses et ce kit sont destinés à être utilisés dans le traitement de la dépendance, plus particulièrement de la dépendance tabagique.

L'invention va être décrite de façon plus détaillée en référence aux exemples suivants qui sont donnés uniquement à titre d'illustration et ne sont pas limitatifs.

### EXEMPLES :

### EXEMPLE 1 : Préparation de solutions pour injection

Des solutions pour injection ont été préparées en dissolvant 20 mg d'un lyophilisat IP 100 de feuilles de tabac commercialisé par la Société Stallergènes dans 2 ml d'eau stérile.

### EXEMPLE 2 :

150 patients souffrant de dépendance tabagique ont été soumis à un questionnaire portant sur les allergies, les risques cardio-vasculaires, le diabète, la sensibilité à l'alcool, aux toxiques chimiques ou aux drogues connues. Chacun des 150 patients a alors reçu une injection sous cutanée (dans l'avant-bras ou la fesse) d'une solution préparée ci-dessus.

10 à 12 jours plus tard, 15 patients ressentant encore l'envie de fumer ont reçu une seconde injection identique à la première.

Le pourcentage de patients n'ayant pas repris de cigarettes au bout de 1 mois, 3 mois, 6 mois et 12 mois est donné ci-après, en termes de taux de succès:

### Taux de succès :

1 MOIS : 74 % dont 69% avec une injection et 5% avec deux injections.
3 MOIS : 61 %
6 MOIS : 57 %
12 MOIS : 53 %

Le pourcentage de patients ayant recommencé à fumer 1 mois après la dernière injection est donné ci-après en termes de taux d'échec:

### Taux d'échec :

1 MOIS : 26% dont 25% avec une injection et 1% avec deux injections.

Le taux d'échec de 26% devrait théoriquement être ramené à 10% en pratiquant systématiquement la deuxième injection. Les résultats à 3, 6, et 12 mois en seraient renforcés d'autant.

Les résultats obtenus sont donc très supérieurs aux produits actuellement sur le marché :
16 à 20% de succès pour les timbres, patchs ou gommes après un an de cure,
20 % de succès pour le Zyban®.

Le traitement est de courte durée et beaucoup moins onéreux que les traitements sur plusieurs mois actuels.

## Revendications

1. Solution stérile aqueuse d'un extrait de feuilles de tabac ayant une teneur en matière sèche de 0,05 à 150 mg/ml d'eau stérile, de préférence de 0,5 à 100 mg/ml d'eau stérile, et plus préférentiellement encore de 1 à 20 mg/ml d'eau stérile.

2. Solution selon la revendication 1, **caractérisée en ce que** ledit extrait de feuilles de tabac est un extrait aqueux de feuilles de tabac.

3. Kit comprenant une seringue, un flacon rempli d'eau stérile et un lyophilisat d'extrait de feuilles de tabac.

4. Kit selon la revendication 3, **caractérisé en ce que** ledit extrait de feuilles de tabac est un extrait aqueux de feuilles de tabac.

5. Solution aqueuse d'un extrait de feuilles de tabac dans de l'eau stérile pour injection, pour son utilisation dans une méthode de traitement de la dépendance tabagique, ladite solution présentant une teneur en matière sèche de 0,05 à 150 mg/ml d'eau stérile, de préférence de 0,5 à 100 mg/ml d'eau stérile, et plus préférentiellement encore de 1 à 20 mg/ml d'eau stérile.

6. Solution pour une utilisation selon la revendication 5, pour une administration par voie sous cutanée.

7. Solution pour une utilisation selon la revendication 5 ou la revendication 6, **caractérisée en ce que** ledit extrait de feuilles de tabac est un extrait aqueux de feuilles de tabac.

8. Solution pour une utilisation selon l'une quelconque des revendications 5-7, **caractérisée en ce que** ledit extrait de feuilles de tabac est lyophilisé.

9. Solution pour une utilisation selon l'une quelconque des revendications 5-7, **caractérisée en ce que** ladite méthode de traitement de la dépendance comprend une injection unique de ladite solution.

10. Solution pour une utilisation selon l'une quelconque des revendications 5-7, **caractérisée en ce que** ladite méthode de traitement de la dépendance comprend au moins deux injections faites à des intervalles de temps de 4 à 30 jours, de préférence 5 à 15 jours, plus préférentiellement encore de 6 à 13 jours.

## Patentansprüche

1. Sterile wässrige Lösung eines Tabakblätterextrakts mit einem Trockenmassegehalt von 0,05 bis 150 mg/ml sterilen Wassers, vorzugsweise von 0,5 bis 100 mg/ml sterilen Wassers, und in noch bevorzugterer Weise von 1 bis 20 mg/ml sterilen Wassers.

2. Lösung nach Anspruch 1, **dadurch gekennzeichnet, dass** der Tabakblätterextrakt ein wässriger Tabakblätterextrakt ist.

3. Set, das eine Spritze, einen mit sterilem Wasser gefüllten Flakon und ein Tabakblätterextrakt-Lyophilisat umfasst.

4. Set nach Anspruch 3, **dadurch gekennzeichnet, dass** der Tabakblätterextrakt ein wässriger Tabakblätterextrakt ist.

5. Wässrige Lösung eines Tabakblätterextrakts in sterilem Wasser zwecks Injektion für ihre Verwendung bei einer Behandlungsmethode der Tabakabhängigkeit, wobei die Lösung einen Trockenmassegehalt von 0,05 bis 150 mg/ml sterilen Wassers, vorzugsweise von 0,5 bis 100 mg/ml sterilen Wassers, und in noch bevorzugterer Weise von 1 bis 20 mg/ml sterilen Wassers aufweist.

6. Lösung für eine Verwendung nach Anspruch 5 für eine subkutane Verabreichung.

7. Lösung für eine Verwendung nach Anspruch 5 oder Anspruch 6, **dadurch gekennzeichnet, dass** der Tabakblätterextrakt ein wässriger Tabakblätterextrakt ist.

8. Lösung für eine Verwendung nach einem der Ansprüche 5 bis 7, **dadurch gekennzeichnet, dass** der Tabakblätterextrakt lyophilisiert ist.

9. Lösung für eine Verwendung nach einem der Ansprüche 5 bis 7, **dadurch gekennzeichnet, dass** die Behandlungsmethode der Abhängigkeit eine einzige Injektion der Lösung umfasst.

10. Lösung für eine Verwendung nach einem der Ansprüche 5 bis 7, **dadurch gekennzeichnet, dass** die Behandlungsmethode der Abhängigkeit mindestens zwei Injektionen in Zeitintervallen von 4 bis 30 Tagen, vorzugsweise von 5 bis 15 Tagen, in noch bevorzugterer Weise von 6 bis 13 Tagen umfasst.

## Claims

1. Sterile aqueous solution of an extract of tobacco leaves having a dry matter content of 0.05 to 150 mg/ml of sterile water, preferably of 0.5 to 100 mg/ml of sterile water, and more preferably of 1 to 20 mg/ml of sterile water.

2. Solution according to claim 1, **characterized in that** said extract of tobacco leaves is an aqueous extract of tobacco leaves.

3. Kit comprising a syringe, a flask of sterile water and a lyophilizate of extract of tobacco leaves.

4. Kit according to claim 3, **characterized in that** said extract of tobacco leaves is an aqueous extract of tobacco leaves.

5. Aqueous solution of an extract of tobacco leaves in sterile water for injection, for use in a method for treating tobacco dependence, said solution having a dry matter content of 0.05 to 150 mg/ml of sterile water, preferably of 0.5 to 100 mg/ml of sterile water, and more preferably of 1 to 20 mg/ml of sterile water.

6. Solution for use according to claim 5, for subcutaneous administration.

7. Solution for use according to claim 5 or claim 6, **characterized in that** said extract of tobacco leaves is an aqueous extract of tobacco leaves.

8. Solution for use according to any one of claims 5-7, **characterized in that** said extract of tobacco leaves is lyophilized.

9. Solution for use according to any one of claims 5-7, **characterized in that** said method for treating dependence comprises a single injection of said solution.

10. Solution for use according to any one of claims 5-7, **characterized in that** said method for treating dependence comprises at least two injections performed at time intervals of 4 to 30 days, preferably of 5 to 15 days, more preferably of 6 to 13 days.
